(19) Europäisches Patentamt
European Patent Office
Office européen des brevets

(11) EP 2 198 852 A1

(12) DEMANDE DE BREVET EUROPEEN

(43) Date de publication:
**23.06.2010 Bulletin 2010/25**

(21) Numéro de dépôt: **09178462.9**

(22) Date de dépôt: **09.12.2009**

(51) Int Cl.:
***A61K 8/92*** *(2006.01)* ***A61K 8/97*** *(2006.01)*
***A61Q 3/02*** *(2006.01)* ***A61K 8/36*** *(2006.01)*

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **17.12.2008 FR 0858694**
**18.02.2009 US 153321 P**

(71) Demandeur: **L'Oréal**
**75008 Paris (FR)**

(72) Inventeur: **Kergosien, Guillaume**
**92370, Chaville (FR)**

(74) Mandataire: **Le Coupanec, Pascale A.M.P.**
**Nony & Associés**
**3, rue de Penthièvre**
**75008 Paris (FR)**

# (54) Vernis à ongles comprenant une huile siccative et un sel métallique

(57) La présente invention a pour objet un vernis à ongles, **caractérisé en ce qu**'il comprend au moins une huile siccative et au moins un sel métallique, et en ce qu'il comprend moins de 5 % en poids de solvant(s) organique(s) volatil(s) par rapport au poids total du vernis à ongles.

La présente invention a également pour objet un procédé cosmétique de maquillage et/ou de soin non thérapeutique des ongles comprenant l'application sur les ongles d'au moins une couche dudit vernis à ongles.

EP 2 198 852 A1

## Description

La présente invention a pour objet un vernis à ongles comprenant au moins une huile siccative et au moins un sel métallique, et comprenant moins de 5 % en poids de solvant(s) organique(s) volatil(s) par rapport au poids total du vernis à ongles.

Cette composition de vernis à ongles peut être appliquée sur les ongles d'êtres humains ou bien encore sur des faux ongles.

La présente invention se rapporte en outre au procédé de maquillage et/ou de soin non thérapeutique des ongles correspondant.

La composition de vernis à ongles, colorée ou transparente, peut être employée comme base pour vernis ou « *base-coat* » en terminologie anglo-saxonne, comme produit de maquillage des ongles, comme composition de finition, encore appelée « top-coat », à appliquer sur le produit de maquillage des ongles ou bien encore comme produit de soin cosmétique des ongles.

Les compositions de vernis à ongles comportent traditionnellement des composés en solution dans des solvants volatils organiques. Ces compositions forment des films après évaporation du solvant.

Cependant, ces compositions présentent l'inconvénient de libérer, lors du séchage, des composés volatils odorants, qui peuvent représenter une gêne pour l'utilisateur ou les personnes environnantes.

Il demeure donc un besoin de disposer de compositions de vernis à ongles comprenant une faible teneur en solvant(s) organique(s) volatil(s), voire exemptes de solvant organique volatil.

Les inventeurs ont découvert qu'il était possible d'obtenir une telle composition de vernis à ongles comprenant moins de 5 % en poids de solvant(s) organique(s) volatil(s) par rapport au poids total de la composition, voire en étant exempte, en utilisant au moins une huile siccative, de préférence au moins une huile de lin raffinée ou soufflée, et au moins un sel métallique, de préférence exempt de cobalt.

Ainsi, selon un de ses aspects, la présente invention a pour objet un vernis à ongles comprenant au moins une huile siccative, de préférence au moins une huile de lin raffinée ou soufflée, au moins un sel métallique, de préférence exempt de cobalt, et comprenant moins de 5 % en poids de solvant(s) organique(s) volatil(s) par rapport au poids total du vernis à ongles, de préférence étant exempt de solvant organique volatil.

Un tel vernis à ongles présente notamment une bonne stabilité dans le temps, et permet la formation d'un film homogène, brillant, et/ou non cassant.

Selon un autre aspect, l'invention a encore pour objet un procédé cosmétique de maquillage et/ou de soin non thérapeutique des ongles comprenant l'application sur les ongles d'au moins une couche d'un vernis à ongles tel que défini précédemment.

Selon encore un autre de ses aspects, l'invention a encore pour objet l'utilisation d'un vernis à ongles tel que défini précédemment pour obtenir, après dépôt sur l'ongle, un film homogène, brillant, et/ou non cassant.

Le vernis à ongles selon l'invention comprend un milieu cosmétiquement acceptable, c'est-à-dire un milieu non toxique et susceptible d'être appliqué sur les matières kératiniques d'êtres humains, en particulier les ongles.

La présente invention concerne encore un support synthétique maquillé comprenant un maquillage obtenu par le procédé de l'invention.

Le vernis à ongles selon l'invention présente une texture liquide ou gélifiée ; il diffère en particulier d'un patch ou article souple comprenant au moins une couche de matériau polymérique et une couche d'adhésif, destiné à être collé et ajusté grâce à ses propriétés de déformabilité, sur l'ongle.

Autrement dit, le vernis à ongles selon l'invention est typiquement appliqué sous forme de couches superposées à la surface des ongles ou des faux ongles à maquiller, par exemple à l'aide d'un pinceau.

Un vernis à ongles selon l'invention comprend en particulier moins de 5 % en poids de solvant(s) organique (s) volatil(s), voire moins de 2 % en poids, voire est exempt de solvant organique volatil.

Par « solvant organique volatil », on entend un solvant organique (ou milieu non aqueux) susceptible de s'évaporer au contact de l'ongle en moins d'une heure, à température ambiante et pression atmosphérique. Un tel solvant organique est un solvant cosmétique volatil, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm de Hg), et de préférence allant de 1,3 Pa à 8000 Pa (0,01 à 60 mm de Hg).

Ces solvants organiques sont notamment ceux classiquement utilisés dans les vernis à ongles. Parmi ces solvants, on peut citer à titre d'exemples, les cétones, comme l'acétone, la méthyl-éthylcétone, la méthyl-isobutyl-cétone ; les éthers de glycol ; les alcools comme l'éthanol, le n-butanol, le n-propanol, l'isopropanol ; les acétates comme l'acétate de butyle, d'éthyle, d'isopropyle, l'acétate de méthoxy-2-éthyle ; les hydrocarbures linéaires ou ramifiés tels que l'hexane ou l'octane ; les hydrocarbures aromatiques tels que le xylène et le toluène ou encore les huiles siliconées volatiles.

## Huiles siccatives

Un vernis à ongles selon l'invention comprend au moins une huile siccative.

Par « huile siccative », on entend désigner une huile qui, lorsque étalée en couche mince puis exposée à l'air, se transforme en pellicule solide.

En particulier, on entend désigner dans le cadre de la présente invention par « huile siccative », les huiles, et de préférence les triglycérides, comportant des doubles liaisons conjuguées, de préférence comportant au moins deux doubles liaisons conjuguées, et de préférence comportant au moins trois doubles liaisons conjuguées.

Les huiles siccatives conformes à l'invention peuvent être d'origine naturelle.

De manière avantageuse, l'huile siccative peut être choisie parmi les huiles végétales siccatives telles que l'huile de lin, l'huile de bois de Chine (ou Canton), l'huile d'oïticica, l'huile de vernonia, l'huile d'oeillette, l'huile de grenade, l'huile de calendula ; les esters de ces huiles végétales ; les résines alkydes obtenues à partir de ces huiles végétales ; et leurs mélanges.

Les résides alkydes sont des polyesters comprenant des chaînes hydrocarbonées d'acides gras, obtenus notamment par polymérisation de polyols et de polyacides ou de leur anhydride correspondant, en présence d'acides gras. Ces acides gras sont présents, notamment sous la forme de triglycérides, dans la majorité des huiles naturelles, telles qu'en particulier les huiles citées précédemment.

L'huile siccative convenant à la mise en oeuvre de la présente invention peut être modifiée par réaction chimique.

En particulier, elle peut être raffinée et/ou partiellement polymérisée. A ce titre, on peut citer les huiles soufflées et les standolies, les huiles maléinisées, époxydées ou cuites.

Selon un mode de réalisation particulier, l'huile siccative de l'invention est distincte d'une huile époxydée.

Selon un mode de réalisation particulier de l'invention, l'huile siccative est une huile de lin raffinée.

Une huile peut être raffinée en particulier en trois étapes successives.

L'huile de lin raffinée selon l'invention peut ainsi résulter d'une étape de dégommage, pour obtenir en particulier une huile démucilaginée, suivie d'une étape de décoloration, en particulier pour la blanchir, puis d'une étape de neutralisation.

Selon un mode particulier de réalisation de l'invention, l'huile siccative de l'invention est une huile de lin modifiée selon au moins l'une des trois étapes mentionnées précédemment, autrement dit qui a subi soit une étape de dégommage, soit une étape de décoloration, soit une étape de neutralisation, soit une succession de ces étapes.

Selon encore un autre mode particulier de l'invention, l'huile siccative de l'invention est une huile de lin modifiée qui a subi deux étapes parmi celles considérées précédemment.

Selon un mode de réalisation particulier de l'invention, l'huile siccative est une huile de lin soufflée.

Le soufflage d'une huile se caractérise notamment par une polymérisation de ladite huile avec l'oxygène de l'air. L'huile soufflée peut être en particulier obtenue en soufflant de l'air au travers de l'huile chauffée.

Selon un mode particulier de réalisation, les compositions cosmétiques de vernis à ongles selon l'invention comprennent de 40 à 99,9 % en poids, notamment de 60 à 99 % en poids, et en particulier de 65 à 97 % en poids d'huile siccative par rapport au poids total de la composition.

## Sels métalliques

Un vernis à ongles conforme à l'invention comprend au moins un sel métallique, et de préférence au moins deux sels métalliques distincts.

L'association d'au moins une huile siccative avec un(des) sel(s) métallique(s) permet notamment lors du séchage de former un film par réticulation avec l'oxygène de l'air.

Le(s) sels métallique(s) accélère(ent) en particulier le séchage des huiles siccatives naturelles.

Avantageusement, le sel métallique utilisé comprend moins de 5 % de cobalt par rapport au poids total du métal dans les sels métalliques, voire est avantageusement exempt de cobalt, en particulier afin d'obtenir un profil toxicologique plus favorable.

Selon un mode de réalisation particulier, le(s) sel(s) métallique(s) conforme(s) à l'invention est(sont) choisi(s) parmi les sels de manganèse, de calcium, de zirconium, de zinc, de strontium, de lithium, de cérium et de vanadium.

Selon un mode de réalisation particulier, le sel métallique est un sel organique. Il peut notamment présenter la formule (I) suivante :

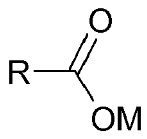
(I)

dans laquelle :

M est choisi parmi le manganèse, le calcium, le zirconium, le zinc, le strontium, le lithium, le cérium, et le vanadium ; et

R est un radical ($C_1$-$C_{25}$)alkyle, linéaire ou ramifié, pouvant éventuellement comporter 1 à 3 insaturations.

Selon un mode particulier, R est un radical ($C_6$-$C_{20}$)alkyle, linéaire ou ramifié, pouvant éventuellement comporter 1 à 2 insaturations.

Selon un mode particulier, le sel métallique peut prendre la forme d'octoate, de linoléate, d'octanoate, d'oléate, de stéarate, de laurate ou de naphténate.

Selon un mode de réalisation particulier, un vernis à ongles conforme à l'invention comprend au moins un sel de manganèse.

Selon un autre mode de réalisation particulier de l'invention, la composition de vernis à ongles selon l'invention comprend plusieurs sels métalliques, en particulier dans le but d'augmenter la vitesse de réticulation de l'huile siccative.

Selon un mode de réalisation particulier de l'invention, les compositions cosmétiques de vernis à ongles selon l'invention comprennent de 0,001 à 2 % en poids, notamment de 0,01 à 1 % en poids, en particulier de 0,1 à 0,5 % en poids de métaux par rapport à l'extrait sec.

Des exemples de produits commerciaux de sels métalliques sont donnés ci-après : Octa-Soligen Manganese 6HS, Octa-Soligen Zirconium 12 HS, et Octa-Soligen Calcium 5 HS, commercialisés par la société OMG BORCHERS GMBH.

L'indication xHS (6HS, 12HS et 5HS) employée ci-dessus, indique que le produit commercial ou matière première contient x pourcent en métal par rapport au poids total du produit commercial ou sel métallique.

Selon un mode particulier de l'invention, un vernis à ongles selon l'invention peut comprendre au moins deux sels métalliques différents choisis parmi les sels tels que définis précédemment.

Selon un autre mode particulier de l'invention, les sels métalliques d'un vernis à ongles de l'invention peuvent comprendre au moins un sel métallique primaire et au moins un sel métallique secondaire, voire peuvent consister en un sel métallique primaire et un sel métallique secondaire.

Dans le cadre de ce mode de réalisation particulier,

- le sel métallique primaire peut être choisi parmi les sels de manganèse, les sels de cérium et les sels de vanadium, et plus particulièrement, est un sel de manganèse ; et
- le sel métallique secondaire peut être choisi parmi les sels de zirconium, de zinc, de lithium et de strontium, et plus particulièrement, est un sel de zirconium.

Dans le cadre de ce mode de réalisation particulier, les sels métalliques d'un vernis à ongles de l'invention peuvent comprendre au moins un sel de manganèse et au moins un sel de zirconium.

Selon encore un autre mode de réalisation préféré de l'invention, un vernis à ongles de l'invention peut comprendre au moins un sel métallique primaire tel que défini précédemment, au moins un sel métallique secondaire tel que défini précédemment, et au moins un sel métallique auxiliaire.

Dans le cadre de ce dernier mode de réalisation particulier, le sel métallique auxiliaire peut être choisi parmi les sels de calcium.

Selon un mode de réalisation particulièrement préféré, les sels métalliques d'un vernis à ongles de l'invention peuvent comprendre au moins un sel de manganèse, au moins un sel de zirconium et au moins un sel de calcium, voire peuvent consister en un sel de manganèse, un sel de zirconium et un sel de calcium.

Dans le cadre de ce mode de réalisation particulièrement préféré, le sel de manganèse peut notamment favoriser le séchage superficiel et le séchage complet du film ; le sel de zirconium peut favoriser le séchage en profondeur, induisant un séchage régulier du film ; et le sel de calcium peut permettre de développer un effet synergétique entre le sel manganèse et le sel de zirconium.

Toujours selon ce mode de réalisation particulièrement préféré, la composition de vernis à ongles de l'invention peut comprendre au moins un sel métallique primaire tel que défini précédemment dans une teneur allant de 0,005 à 0,1 % en poids de métal par rapport à l'extrait sec, notamment de 0,01 à 0,05 % en poids de métal par rapport à l'extrait

sec. Elle peut en outre comprendre au moins un sel métallique secondaire tel que défini précédemment dans une teneur allant de 0,005 à 0,8 % en poids de métal par rapport à l'extrait sec, notamment de 0,03 à 0,5 % en poids de métal par rapport à l'extrait sec. Elle peut enfin de plus comprendre au moins un sel métallique auxiliaire tel que défini précédemment dans une teneur allant de 0,03 à 0,3 % en poids de métal par rapport à l'extrait sec.

## CARACTERISATION DE L'EXTRAIT SEC

La teneur en matière sèche, communément appelée « extrait sec », c'est-à-dire la teneur en matière non volatile, peut être mesurée de différentes manières. On peut citer par exemple les méthodes par séchage à l'étuve, les méthodes par séchage par exposition à un rayonnement infrarouge.

De préférence, la quantité de matière sèche des compositions de vernis à ongles selon l'invention est mesurée par échauffement de l'échantillon par des rayons infrarouges de 2 μm à 3,5 μm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant :

On étale environ 1g de la composition sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :

$$\text{Extrait Sec} = 100 \text{ x } (\text{masse sèche} / \text{masse humide}).$$

Les compositions selon l'invention se caractérisent par une teneur en matière sèche supérieure ou égale à 95 %, en poids, plus particulièrement supérieure à 99 % en poids.

Dans le cas particulier où la composition est totalement exempte de solvant organique volatil, l'extrait sec peut être assimilable au poids de la composition totale du vernis à ongles.

## Agent filmogène

Un vernis à ongles selon l'invention peut comprendre en outre un agent filmogène. L'agent filmogène est généralement un polymère filmogène.

Par « polymère filmogène », on désigne au sens de la présente invention, un polymère apte à former à lui seul ou en présence d'un agent auxiliaire de filmification, un film isolable, notamment continu et adhérent, sur un support, notamment sur les ongles.

En particulier, un polymère filmogène selon l'invention est distinct des huiles siccatives telles que décrites précédemment.

Dans la composition, on peut utiliser un seul polymère filmogène ou un mélange de polymères filmogènes.

Ce polymère filmogène peut être choisi dans le groupe constitué par les polymères synthétiques, de type radicalaire ou de type polycondensat, les polymères d'origine naturelle et leurs mélanges.

Un polymère filmogène convenant à l'invention peut être choisi en particulier parmi :

- les dérivés de polysaccharides, tels que les dérivés de cellulose ou de gomme de guar. Un dérivé de polysaccharides convenant à l'invention peut être un ester ou alkyléther de polysaccharide.

Par « ester ou alkyléther de polysaccharide », on désigne un polysaccharide formé de motifs répétitifs comportant au moins deux cycles identiques ou différents et présentant un degré de substitution par unité de saccharide compris entre 1,9 et 3 de préférence compris entre 2,2 et 2,9, et plus particulièrement entre 2,4 et 2,8. On désigne par substitution la fonctionnalisation des groupements hydroxyles en fonctions esters et/ou alkyléthers, et/ou la fonctionnalisation des groupements carboxyliques en fonctions esters.

Autrement dit, il peut s'agir d'un polysaccharide, partiellement ou totalement substitué par des groupements esters et/ou alkyléthers. De préférence, les groupements hydroxyles peuvent être substitués par des fonctions ester et/ou alkyléther en $C_2$-$C_4$.

On peut citer en particulier les esters de cellulose, tels que l'acétate de cellulose, les acétobutyrates de cellulose

ou les acétopropianates de cellulose ; les alkyléthers de cellulose comme les éthylcelluloses, et les éthylguars ;

- les polymères synthétiques tels que les polyuréthanes, les polymères acryliques, les polymères vinyliques, les polyvinylbutyrals, les résines alkydes et les résines cétone/aldéhyde, les résines issues des produits de condensation d'aldéhydes, telles que les résines arylsulfonamide formaldéhyde comme la résine toluène sulfonamide formaldéhyde, les résines aryl-sulfonamide époxy ou encore les résines éthyl tosylamide ;
- les polymères d'origine naturelle, tels que les résines végétales telles que les dammars, l'élémi, les copals, le benjoin ; les gommes telles que la gomme laque, la gomme sandaraque et la gomme mastic.

Comme polymère filmogène, on peut notamment utiliser les résines toluène sulfonamide formaldéhyde « Ketjentflex MS80 » de la société AKZO ou « Santolite MHP », « Santolite MS 80 » de la société FACONNIER ou « RESIMPOL 80 » de la société PAN AMERICANA, la résine alkyde « BECKOSOL ODE 230-70-E » de la société DAINIPPON, la résine acrylique « ACRYLOID B66 » de la société ROHM & HAAS, la résine polyuréthane « TRIXENE PR 4127 » de la société BAXENDEN, la résine acétophénone/formaldéhyde commercialisée sous la référence Synthetic Resin SK par Degussa.

Selon un mode de réalisation préféré, l'agent filmogène est choisi parmi les gommes et les résines végétales telles que la gomme laque, la gomme sandaraque, les dammars, l'élémi, les copals, le benjoin, et la gomme mastic.

Par exemple, on peut utiliser comme polymère filmogène la gomme élémi distillée, purifiée et concassée commercialisée par la société EMIGA.

Selon un mode de réalisation particulier, un vernis à ongles selon l'invention comprend moins de 30 % en poids, notamment moins de 20 % en poids, en particulier moins de 10 % en poids d'agent filmogène par rapport au poids total de la composition.

Selon un mode de réalisation préféré, une composition de vernis à ongles selon l'invention comprend moins de 5 % en poids en matière sèche de nitrocellulose, par rapport au poids total de la composition, de préférence moins de 3 % en poids, de préférence moins de 1,5 % poids, de préférence moins de 1 % en poids.

Selon un mode de réalisation particulièrement préféré, la composition de vernis à ongles de l'invention est totalement dépourvue de nitrocellulose.

Selon un mode de réalisation particulier de l'invention, un vernis à ongles selon l'invention comprend au moins une huile de lin soufflée et au moins des sels de manganèse, de zirconium et de calcium.

Selon encore un mode particulier de l'invention, un vernis à ongles selon l'invention comprend en outre de la gomme élémi.

## Agent auxiliaire de filmification

Pour améliorer les propriétés filmogènes de la composition de vernis à ongles un agent auxiliaire de filmification peut être prévu.

Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).

Ainsi, la composition peut comprendre, en outre, au moins un agent plastifiant et/ou un agent de coalescence. En particulier, on peut citer, seuls ou en mélange, les plastifiants et agents de coalescence usuels, tels que :

- des esters d'acides, notamment carboxyliques, tels que des citrates, des phtalates, des adipates, des carbonates, de tartrates, des phosphates, des sébaçates ;
- des dérivés oxyéthylénés, tels que les huiles oxyéthylénées, notamment des huiles végétales, telles que l'huile de ricin ;
- les huiles d'origine naturelle, en particulier non siccatives, choisies parmi des huiles comprenant au moins un acide gras choisi parmi l'acide caprylique, l'acide caprique, l'acide laurique, l'acide myristique, l'acide palmitique, l'acide stéarique, l'acide oléique, l'acide ricinoléique, l'acide linoléique, l'acide linolénique, l'acide arachidique, l'acide gadoléique, l'acide béhénique, l'acide érucique, l'acide brassidique, l'acide cétoléique, l'acide lignocérique, l'acide nervonique. En particulier, ces huiles sont choisies parmi les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées. Ces huiles sont notamment des triglycérides héptanoïques ou octanoïques, les huiles d'arachide, de babassu, de noix de coco, de pépins de raisin, de coton, de maïs, de germes de maïs, de graines de moutarde, de palme, de colza, de sésame, de soja, de tournesol, de germe de blé, de canola, d'abricot, de mangue, de karité, d'avocat, d'olive, d'amande douce, d'amande, de pêche, de noix, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat, de beurre de karité ou encore les

triglycérides des acides caprylique/caprique, et
- leurs mélanges.

Le type et la quantité d'agent(s) plastifiant(s) et/ou de coalescence peuvent être choisis par l'homme du métier sur la base de ses connaissances générales.
Par exemple, la teneur en agent plastifiant et/ou de coalescence peut aller de 0,01 % à 20 % et en particulier de 0,5 % à 10 % en poids par rapport au poids total de la composition.

**Agent gélifiant**

La composition de vernis à ongles selon l'invention peut comprendre en outre un agent gélifiant.
Cet agent gélifiant peut en particulier être choisi parmi : les silices hydrophobes, telles que celles décrites dans le document EP-A-0 898 960, et par exemple commercialisées sous les références « AEROSIL R812® » par la société Degussa, « CAB-O-SIL TS-530®«, « CAB-O-SIL TS-610® », « CAB-O-SIL TS-720® »par la société Cabot, « AEROSIL R972® », « AEROSIL R974® » par la société Degussa ; les argiles telles que la montmorillonite, les argiles modifiées telles que les bentones par exemple, l'hectorite stéaralkonium, la bentonite stéaralkonium, les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-0 898 958.
La proportion totale en agent(s) gélifiant(s) dans les compositions selon l'invention peut aller de à 0,01 à 15 % en poids, par rapport au poids total de la composition, de préférence de 0,5 à 15 % et mieux de 0,5 à 10 % en poids.

**Matière colorante**

La composition de vernis à ongles selon l'invention peut, en outre, comprendre une ou des matières colorantes choisies parmi les colorants solubles et les matières colorantes pulvérulentes comme les pigments, les nacres, et les paillettes bien connues de l'homme du métier.
Les matières colorantes peuvent être présentes, dans la composition, en une teneur allant de 0,01 % à 50 % en poids, par rapport au poids de la composition, de préférence de 0,01 % à 30 % en poids.
Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.
Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.
On peut également citer les pigments à effet tels les particules comportant un substrat organique ou minéral, naturel ou synthétique, par exemple le verre, les résines acrylique, polyester, polyuréthane, polyéthylène téréphtalate, les céramiques, les alumines et recouvert ou non de substances métalliques comme l'aluminium, l'or, le cuivre, le bronze, ou d'oxydes métalliques comme le dioxyde de titane, l'oxyde de fer, l'oxyde de chrome, de pigments minéraux ou organiques et leurs mélanges.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
On peut aussi utiliser des pigments à propriétés goniochromatiques, notamment à cristaux liquides ou multicouche.
Les colorants sont, par exemple, le rouge Soudan, le DC Red 17, le DC Green 6, le β-carotène, l'huile de soja, le brun Soudan, le DC Yellow 11, le DC Violet 2, le DC Orange 5, le jaune quinoléine.
La matière colorante peut également être choisie parmi les azurants optiques.
La composition peut comprendre, en outre des fibres éventuellement enrobées d'azurant optiques.

**Charge**

La composition selon l'invention peut comprendre en outre une ou plusieurs charges, notamment en une teneur allant de 0,01 % à 50 % en poids, par rapport au poids total de la composition, de préférence allant de 0,01 % à 30 %

en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

## Additifs

La composition peut comprendre, en outre, d'autres ingrédients utilisés couramment dans les compositions cosmétiques et connus de l'homme du métier comme étant susceptibles d'être incorporés dans une composition de vernis à ongles.

De tels ingrédients peuvent être choisis parmi les huiles, les cires, les agents antiradicaux libres, les agents d'étalement, les agents mouillants, les agents dispersants, les anti-mousses, les conservateurs, les tensioactifs, les parfums, les neutralisants, les stabilisants, les antioxydants, les actifs pouvant être choisis parmi les huiles essentielles, les filtres UV/solaires, les agents hydratants, les vitamines, les protéines, les céramides, les extraits végétaux , etc; et leurs mélanges.

Les compositions selon l'invention peuvent comprendre en outre à titre d'actifs, des agents de soin des ongles tels que des agents durcissants pour matières kératiniques, des actifs agissant sur la croissance de l'ongle comme par exemple le méthyl sulfonyle méthane et/ou des actifs pour traiter des affections diverses localisées au niveau de l'ongle, comme par exemple l'onichomycose.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant : i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

L'invention est illustrée plus en détail dans les exemples suivants qui sont présentés à titre illustratif et non limitatif de l'invention.

Sauf indication contraire, les quantités sont données en pourcentage en poids par rapport au poids total de la composition.

## EXEMPLES

### Exemple 1

| | |
|---|---|
| Huile de lin soufflée« LIN SOUFFLE CV 5P » (1) | 96% |
| Octoate de manganèse (2) | 0,6% |

(suite)

| | |
|---|---|
| Octoate de zirconium (3) | 1,8 % |
| Octoate de calcium, neutre(4) | 1,6 % |
| (1) commercialisée par la société Novance<br>(2) Octa-Soligen Manganese 6HS commercialisé par la société OMG BORCHERS GMBH<br>(3) Octa-Soligen Zirconium 12 HS commercialisé par la société OMG BORCHERS GMBH<br>(4) Octa-Soligen Calcium 5 HS commercialisé par la société OMG BORCHERS GMBH | |

Exemple référence (sans sels métalliques)

| | |
|---|---|
| Huile de lin soufflée « LIN SOUFFLE CV 5P » (1) | 100 % |

Mode opératoire :

Des films de ces deux compositions sont enduits à une épaisseur de 50 μm humide sur carte de contraste.

Résultat :

Après 7 heures de séchage à 30 °C, la composition de l'exemple 1 forme un film solide, alors que la composition de référence est encore liquide.

**Exemple 2**

| | |
|---|---|
| Huile de lin soufflée « LIN SOUFFLE CV 5P » (1) | 86,4 % |
| Gomme élémi distillée purifiée concassée(5) | 9,6% |
| Octoate de manganèse (2) | 0,6% |
| Octoate de zirconium(3) | 1,8 % |
| Octoate de calcium, neutre(4) | 1,6 % |
| (5) commercialisée par la société Emiga | |

Mode opératoire :

Dans un premier temps, l'huile de lin soufflée et la gomme élémi sont mélangées.
Le mélange est agité jusqu'à solubilisation complète de la gomme élémi. Les sels métalliques sont ensuite ajoutés.
Un film de la composition est enduit à une épaisseur de 50 μm humide sur carte de contraste.

Résultat :

Après séchage, la composition forme un film solide.

**Revendications**

1. Vernis à ongles, **caractérisé en ce qu'**il comprend au moins une huile siccative et au moins un sel métallique, et

**en ce qu'**il comprend moins de 5 % en poids de solvant(s) organique(s) volatil(s) par rapport au poids total du vernis à ongles.

2. Vernis à ongles selon la revendication 1, **caractérisé en ce que** l'huile siccative est choisie parmi l'huile de lin, l'huile de bois de Chine, l'huile d'oïticica, l'huile de vernonia, l'huile d'oeillette, l'huile de grenade, l'huile de calendula ; les esters de ces huiles végétales ; les résines alkydes obtenues à partir de ces huiles végétales, et leurs mélanges, ladite huile siccative pouvant éventuellement être modifiée par réaction chimique.

3. Vernis à ongles selon l'une quelconque des revendications précédentes, **caractérisé en ce que** l'huile siccative est l'huile de lin raffinée ou soufflée.

4. Vernis à ongles selon l'une quelconque des revendications précédentes, **caractérisé en ce que** l'huile siccative est présente en une teneur allant de 40 à 99,9 % en poids, notamment de 60 à 99 % en poids, en particulier de 65 à 97 % en poids par rapport au poids total du vernis à ongles.

5. Vernis à ongles selon l'une quelconque des revendications précédentes, **caractérisé en ce que** le sel métallique comprend au moins un sel métallique choisi parmi les sels de manganèse, de calcium, de zirconium, de zinc, de strontium, de lithium, de cérium et de vanadium.

6. Vernis à ongles selon la revendication 5, **caractérisé en ce que** le sel métallique est un sel organique présentant la formule (I) suivante :

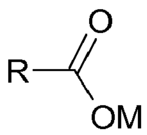
(I)

dans laquelle :

M est choisi parmi les métaux tels que définis dans la revendication précédente ; et
R est un radical ($C_1$-$C_{25}$)alkyle, linéaire ou ramifié, pouvant éventuellement comporter 1 à 3 insaturations, et plus particulièrement un radical ($C_6$-$C_{20}$)alkyle, linéaire ou ramifié, pouvant éventuellement comporter 1 à 2 insaturations ;
et en particulier, prend la forme d'octoate, de linoléate, d'octanoate, d'oléate, de stéarate, de laurate ou de naphténate.

7. Vernis à ongles selon l'une quelconque des revendications précédentes, **caractérisé en ce qu'**il comprend au moins un sel de manganèse.

8. Vernis à ongles selon l'une quelconque des revendications précédentes, **caractérisé en ce que** le sel métallique comprend au moins deux sels métalliques différents choisis parmi les sels tels que définis en revendications 5 ou 6.

9. Vernis à ongles selon l'une quelconque des revendications précédentes, **caractérisé en ce que** le sel métallique comprend au moins un sel de manganèse et au moins un sel de zirconium.

10. Vernis à ongles selon l'une quelconque des revendications précédentes, **caractérisé en ce que** le sel métallique comprend au moins un sel de manganèse, au moins un sel de zirconium et au moins un sel de calcium.

11. Vernis à ongles selon l'une quelconque des revendications précédentes, **caractérisé en ce que** le(s) sel(s) métallique(s) est(sont) présent(s) en une teneur allant de 0,001 à 2 % en poids, notamment de 0,01 à 1 % en poids et en particulier de 0,1 à 0,5 % en poids par rapport à l'extrait sec.

12. Vernis à ongles selon l'une quelconque des revendications précédentes, **caractérisé en ce qu'**il comprend moins de 2 % en poids de solvant(s) organique(s) volatil(s) par rapport au poids total du vernis à ongles, voire est exempt de solvant organique volatil.

**13.** Vernis à ongles selon l'une quelconque des revendications précédentes, **caractérisé en ce qu'**il comprend en outre au moins un agent filmogène, en particulier choisi parmi les gommes et les résines végétales, telles que la gomme laque, la gomme sandaraque, les dammars, l'élémi, les copals, le benjoin et la gomme mastic, et en particulier est la gomme élémi.

**14.** Vernis à ongles selon l'une quelconque des revendications précédentes, **caractérisé en ce qu'**il comprend moins de 5 % en poids en matière sèche de nitrocellulose, par rapport au poids total du vernis à ongles, de préférence moins de 3 % en poids, de préférence moins de 1,5 % poids, de préférence moins de 1 % en poids, voire est totalement dépourvu de nitrocellulose.

**15.** Procédé cosmétique de maquillage et/ou de soin non thérapeutique des ongles comprenant l'application sur les ongles d'au moins une couche de vernis à ongles selon l'une quelconque des revendications précédentes.

Europäisches Patentamt
European Patent Office
Office européen des brevets

# RAPPORT DE RECHERCHE EUROPEENNE

Numéro de la demande
EP 09 17 8462

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | EP 0 645 134 A (OREAL [FR]) 29 mars 1995 (1995-03-29) * tableaux I,II * * revendications 1-10 * | 1-15 | INV. A61K8/92 A61K8/97 A61Q3/02 A61K8/36 |
| X | "Huile pour meubles de jardin" , [Online] 18 avril 2002 (2002-04-18), XP002544537 Extrait de l'Internet: URL:http://www.biofa.de/fra/produit/fichtec/ft3752.pdf> [extrait le 2009-09-04] | 1,2,5-9, 12-15 | |
| Y | * le document en entier * | 1-15 | |
| A | ULRICH POTH: "Drying Oils and Related Products" ULLMANN'S ENCYCLOPEDIA OF INDUSTRIAL CHEMISTRY,, 15 juin 2001 (2001-06-15), pages 1-15, XP002544113 * alinéa [07.1] - alinéa [07.3] * | 1-15 | |

DOMAINES TECHNIQUES RECHERCHES (IPC)
A61K
A61Q

Le présent rapport a été établi pour toutes les revendications

1

| Lieu de la recherche | Date d'achèvement de la recherche | Examinateur |
|---|---|---|
| Munich | 22 avril 2010 | Cismaru, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 09 17 8462

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-04-2010

| Document brevet cité au rapport de recherche | Date de publication | Membre(s) de la famille de brevet(s) | Date de publication |
|---|---|---|---|
| EP 0645134 A | 29-03-1995 | AT 171859 T | 15-10-1998 |
| | | CA 2132398 A1 | 29-03-1995 |
| | | DE 69413773 D1 | 12-11-1998 |
| | | DE 69413773 T2 | 04-03-1999 |
| | | ES 2125422 T3 | 01-03-1999 |
| | | FR 2710524 A1 | 07-04-1995 |
| | | JP 2573807 B2 | 22-01-1997 |
| | | JP 7173035 A | 11-07-1995 |
| | | US 5578297 A | 26-11-1996 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 0898960 A
- EP 0898958 A